# EUROPEAN PATENT APPLICATION

(11) **EP 4 477 261 A1**
(43) Date of publication of application: **18.12.2024**
(21) Application number: 24180498.8
(22) Date of filing: 06.06.2024
(51) Int. Cl.: A61N 5/10

(54) **HYBRID CONTINUOUS POSITIVE PRESSURE AND SURFACE IMAGING SYSTEM AND METHOD**

(30) Priority: 14.06.2023 EP 23179272
(71) Applicant: Vision RT Limited, London N3 2JU (GB); Sheba Impact Ltd., 526501 Ramat Gan (IL)
(72) Inventor: HALE, Gideon Matthew, London, N3 2JU (GB); SMITH, Norman Ronald, London, N3 2JU (GB); SYMON, Zvi, 526501 Ramat Gan (IL); DUBINSKI, Sergey, 5265601 Ramat Gan (IL); STRAWBRIDGE, Benjamin, London, N3 2JU (GB)
(74) Representative: Demant

(57) **Abstract**

The present disclosure relates to a hybrid continuous positive pressure and surface imaging system for use in a radiotherapy system, comprising: a positive pressure unit adapted to apply a continuous positive pressure to lungs of a patient during radiotherapy; a surface camera system configured to continuously, or at intervals, capture body surface images of the patient; and a processing unit configured to continuously, or at intervals, model chest and/or abdominal movement of the patient in response to the applied continuous positive pressure based on the body surface images. The disclosure further relates to a computer-implemented method of providing real-time feedback to a positive pressure unit.

## Description

The present disclosure relates to a hybrid continuous positive pressure and surface imaging system for use in a radiotherapy system. The disclosure further relates to a computer-implemented method of providing real-time feedback to a positive pressure unit.

### Background

Radiotherapy is a therapy using ionizing radiation, generally provided as part of cancer treatment to control or kill malignant cells and normally delivered by a linear accelerator. Radiation therapy may be curative in a number of types of cancer if they are localized to one area of the body. Radiation in a radiation beam does not distinguish between healthy cells and malignant cells and may therefore damage healthy cells, as well as malignant cells. As a result, radiation beams, generally, have to be accurately configured and aimed at a target site of a malignancy to concentrate deposited radiant energy at the malignancy, and minimize deposition of radiant energy to surrounding healthy tissue.

A conventional procedure for depositing radiant energy by a therapeutic radiation beam to a malignancy exhibiting motion during irradiation may involve forming the beam sufficiently large so that the malignancy remains within the beam cross section throughout the irradiation process. Respiration-induced tumor motion is a challenge for radiation treatment of tumors in the thorax. One strategy for addressing this challenge is modelling the tumor trajectory and creating an Internal Target Volume (ITV), which allows the patient to breathe freely during treatment. However, this approach is associated with larger volumes of normal lung tissue receiving ablative doses of radiation compared to other motion management strategies.

More recently, Continuous Positive Airway Pressure (CPAP) has been studied as an alternative strategy to reduce tumor motion during radiotherapy. CPAP was originally developed for other purposes, such as a treatment for obstructive sleep apnea and as a non-invasive alternative to intubation and other invasive techniques used in conditions such as pulmonary edema, but has shown to be efficient in reducing tumor motion during radiotherapy. It may be used in combination with other techniques for addressing respiration-induced tumor motion. It has a particularly powerful role in heavy particle radiation of tumors in the chest, where movement mat result in deposition of dose inappropriately. However, there are numerous challenges associated with this technique as well. One example is determining an optimal CPAP pressure for a specific patient, which is a difficult and time-consuming task. Anxiety may also have a major impact on respiratory pattern.

### Summary

The present disclosure relates to a hybrid continuous positive pressure and surface imaging system for use in a radiotherapy system, the system comprising:
a positive pressure unit (PPU) adapted to apply a continuous positive pressure to lungs of a patient during radiotherapy;
a surface camera system configured to continuously, or at intervals, capture body surface images of the patient; and
a processing unit configured to continuously, or at intervals, model chest and/or abdominal movement of the patient in response to the applied continuous positive pressure based on the body surface images.

The inventors have realized that by using a surface camera system to continuously, or at intervals, capture body surface images of the patient and continuously, or at intervals, modelling chest and/or abdominal movement, a system with, preferably, substantially real-time feedback to the PPU is provided. This has several advantages and possible applications. Determining an optimal CPAP pressure for a specific patient may be a difficult and time-consuming task. The presently disclosed system may allow for a CPAP training process outside the radiotherapy room, prior to the radiotherapy treatment, wherein only the PPU, surface camera system and processing unit are needed.

Radiotherapy treatment typically involves a planning or simulation process. Simulation may be performed in a simulation room, which may be the same as the treatment room. The simulation may include, for example, scanning, positioning of the patient and alignment with the radiotherapy beam(s), and preparation of a complete radiotherapy session, wherein doses are calculated and modelling of a target and organs at risk is performed. The presently disclosed hybrid continuous positive pressure and surface imaging system may be used as an integral part of the radiotherapy system but also independently outside the radiotherapy room prior to the radiotherapy session.

The surface camera system and processing unit configured to continuously, or at intervals, model chest and/or abdominal movement of the patient provides a system that can be used to train the patient to reliably reproduce a stable shallow breathing pattern with a limited tidal volume and limited chest wall and/or abdominal movement to achieve the same conditions during both simulation and treatment. The system may provide a signal representative of chest wall/abdominal movement that is sensitive to small changes in CPAP pressure. The effectiveness of the PPU to maintain chest position throughout the breathing cycle can be determined by the hybrid continuous positive pressure and surface imaging system. The preferably substantially real-time feedback mechanism may allow an operator to adjust the positive pressure to a point where chest movement is within predefined limits. This may, according to one embodiment, be done by automatically increasing the pressure in a controlled fashion over a defined ramp period until chest movement is within predefined limits or the pressure reaches a predefined limit.

The system may also be used to detect and monitor paradoxical and unpredictable increases in chest wall and abdominal movements in anxious patients. A further application of the hybrid continuous positive pressure and surface imaging system may be a configuration in which the system provides feedback for synchronization of the radiation beam and/or discontinues operation of the radiation beam upon a deviation of height, shape or movement of the chest and/or abdomen of the patient with respect to an expected height, shape or movement of the chest and/or abdomen of the patient or a deviation in surface caused by the change in lung volume.

The positive pressure unit may, typically, comprise an air pump unit, a flow tube and a control unit configured to control the positive pressure unit. The positive pressure unit may further comprise a power source, such as a rechargeable battery, and a wireless communication unit for communication, directly or through a further unit, with the surface camera system. The processing unit may be located in any of the positive pressure unit and the surface camera system, or in an additional unit. The positive pressure unit may be configured to flatten and reduce motion of the diaphragm during respiration, and expand the lungs and chest cavity.

The surface camera system configured to continuously, or at intervals, capture body surface images of the patient may comprise one or more three-dimensional camera(s) and/or one or more two-dimensional camera(s). The processing unit may be configured to continuously, or at intervals, model chest and/or abdominal movement of the patient based on the body surface images from the camera(s). There are several possible techniques and camera systems that can be used as surface camera system, including systems provided by Vision RT and described in a number of earlier patents and patent application, including US7889906, US7348974, US8135201, US2015/062303, US2015/0265852, US2020/0184625, US 10,441,818, US 2016/0256710, the disclosure of which is incorporated herein by reference.

In a non-limiting example, a three-dimensional model of the surface of a patient is generated utilising a number of stereoscopic video images of at least a chest and/or abdominal region of the patient. The video images are processed to generate a three-dimensional model of the chest and/or abdominal region, which is rapidly updated to account for changes in the movement of the chest and/or abdominal region.

In order to enable the surface model of a patient to be updated as rapidly as possible, a number of techniques may be utilised. In one embodiment there is provided an image processing method comprising the steps of: obtaining stereoscopic images of an individual; generating a surface model of said individual utilising said stereoscopic images; receiving further stereoscopic images of said individual and generating a further model of said individual from said further stereoscopic images and said previous model of said individual.

According to one non-limiting embodiment, a speckled pattern of light is projected onto the surface of the chest and/or abdominal region of the patient to facilitate identification of corresponding portions of the surface of a patient captured from different viewpoints. Images of a patient are obtained and processed together with data identifying the relative locations of the cameras capturing the images to identify 3D positions of a large number of points corresponding to points on the surface of a patient. Another example of a surface camera system monitors the position of patients undergoing radiotherapy by projecting structured light (e.g. laser light) in the form of a line or grid pattern or other predefined pattern onto the surface of a patient and generating a model of the surface on a patient being monitored on the basis of the appearance of the projected pattern in captured images. Based on the model of the surface, which may be a surface of the chest and/or abdominal region of the patient, movement of the chest and/or abdominal region of the patient can be continuously modeled.

The disclosure further relates to a computer-implemented method of providing real-time feedback to a positive pressure unit, the method comprising the steps of:
acquiring continuous positive pressure data from continuous positive pressure applied to lungs of a patient during radiotherapy;
continuously, or at intervals, capturing body surface images of the patient using a surface camera system; and
continuously, or at intervals, modelling chest and/or abdominal movement of the patient in response to the applied continuous positive pressure based on the body surface images.

As would be understood by a person skilled in the art, the presently disclosed computer-implemented method of providing real-time feedback to a positive pressure unit may be performed using any embodiment of the presently disclosed hybrid continuous positive pressure and surface imaging system, and vice versa.

### Description of drawings

The invention will in the following be described with reference to the accompanying drawings. The drawings are examples of embodiments and not limiting to the presently disclosed hybrid continuous positive pressure and surface imaging system and computer-implemented method of providing real-time feedback to a positive pressure unit.
**Fig. 1** shows one embodiment of the presently disclosed hybrid continuous positive pressure and surface imaging system for use in a radiotherapy system.
**Fig. 2** shows a flow chart of a method according to embodiments of the presently disclosed computer-implemented method of providing real-time feedback to a positive pressure unit.
**Fig. 3** shows a front perspective of an embodiment of a surface camera system.
**Fig. 4** shows a schematic block diagram of an embodiment of a processing unit of the presently disclosed hybrid continuous positive pressure and surface imaging system.

### Detailed description

The present disclosure relates to a hybrid continuous positive pressure and surface imaging system for use in a radiotherapy system, the system comprising:
a positive pressure unit (PPU) adapted to apply a continuous positive pressure to lungs of a patient during radiotherapy;
a surface camera system configured to continuously capture body surface images of the patient; and
a processing unit configured to continuously model chest and/or abdominal movement of the patient in response to the applied continuous positive pressure based on the body surface images.

Descriptions of embodiments of the presently disclosed hybrid continuous positive pressure and surface imaging system are provided by way of example and are not intended to limit the scope of the invention. The described embodiments comprise different features, not all of which are necessarily required in all embodiments of the invention. Some embodiments utilize only some of the features or possible combinations of the features. Variations of embodiments of the invention that are described, and embodiments of the invention comprising different combinations of features noted in the described embodiments, will occur to persons skilled in the art.

By using a surface camera system to continuously capture body surface images of the patient and continuously modelling chest and/or abdominal movement, a system with real-time feedback to the PPU may be obtained. This has several advantages and possible applications. For example, it allows for use of a portable PPU. Determining an optimal CPAP pressure for a specific patient may be a difficult and time-consuming task. The presently disclosed system may allow for a CPAP training or preparation process outside the radiotherapy room, prior to the radiotherapy treatment, wherein only the PPU, surface camera system and processing unit are needed. A portable PPU can remain in use after CPAP training or preparation process so that controlled breathing is maintained. The device can be carried or wheeled between the radiotherapy room and a second location or room.

The inventors have realized that by using a surface camera system to continuously capture body surface images of the patient and continuously modelling chest and/or abdominal movement, a system with, preferably, substantially real-time feedback to the PPU is provided. This has several advantages and possible applications. Determining an optimal CPAP pressure for a specific patient may be a difficult and time-consuming task. The presently disclosed system may allow for a CPAP training process outside the radiotherapy room, prior to the radiotherapy treatment, wherein only the PPU, surface camera system and processing unit are needed.

The 'continuously' shall be construed broadly to include receiving series of images and modelling at any suitable rate. It is in this regard, as an example, well-known that a series of still images at a given rate produces the illusion of a moving image. This can be translated to the presently disclosed hybrid continuous positive pressure and surface imaging system and method in the sense that the surface camera system may be configured to capture body surface images of the patient at a certain rate.

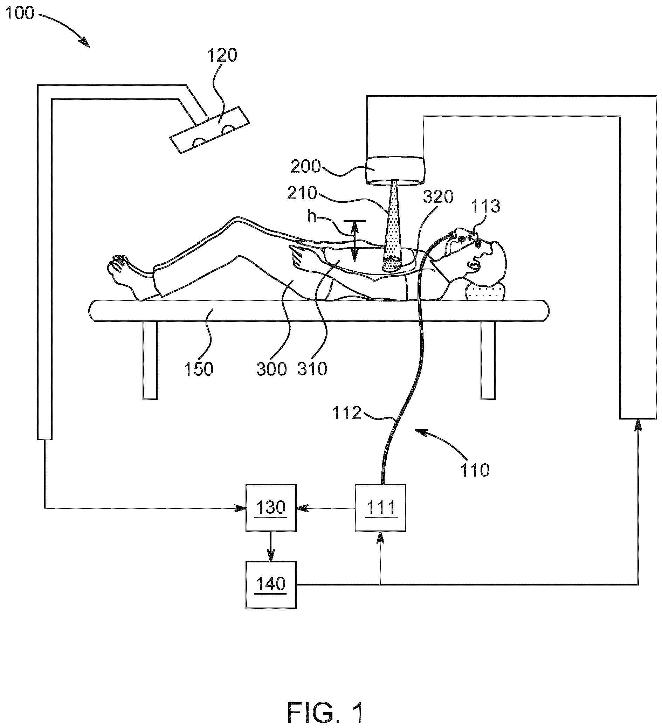

Fig. 1 shows a non-limiting example of hybrid continuous positive pressure and surface imaging system (100) for use in a radiotherapy system (200). The radiotherapy system (200) may be any suitable radiotherapy system and may typically include a radiation beam generator, such as a gated radiation beam generator, typically comprising a linear particle accelerator adapted to generate a radiation beam for irradiating a target volume. The gated radiation beam generator is typically an external device or system. The radiotherapy system may further comprise a controller for controlling the beam generator. The radiotherapy system may be a conventional two-dimensional beam system but could also be, for example, a three-dimensional beam system, an intensity-modulated radiation therapy IMRT system or a volumetric modulated arc therapy VMAT system or heavy particle beam system, such as a proton or carbon ion beam based system. In the example of fig. 1, a radiation beam 210 is generated towards the target 320, which may be a tumor in the patient 300 positioned on the couch 150. A positive pressure unit 110 is configured to apply a continuous positive pressure to lungs of the patient 300 during the radiotherapy treatment. The positive pressure unit 110 comprises an air pump unit 111 and a flow tube 112 connected to the airways of the patient 300 through a face mask 113. A surface camera system 120 is configured to continuously capture body surface images of the patient 300, in particular surface images of the chest and/or abdominal region 310 of the patient 300. A processing unit 130 is configured to continuously model chest and/or abdominal movement of the patient 300 in response to the applied continuous positive pressure based on the body surface images obtained from the surface camera system 120. A control unit 140 is configured to control the positive pressure unit 110 and/or the radiotherapy system 200.

The processing unit is configured to continuously extract an amplitude metric translatable to chest and/or abdominal movement. The amplitude metric may be any suitable metric, such as a change of height of the chest wall or abdomen of the patient. The height of the chest wall may be understood as a component perpendicular to the chest surface of the patient. The height is indicated 'h' in fig. 1. The height of the abdomen may be understood as a component perpendicular to the abdominal surface of the patient.

The processing unit may be configured to continuously model chest and/or abdominal movement in six degrees of freedom (6DOF) based on images form the surface camera system. `6DOF' generally refers to the freedom of movement of a rigid body in three-dimensional space. Specifically, the body is free to change position as forward/backward (surge), up/down (heave), left/right (sway) translation in three perpendicular axes, combined with changes in orientation through rotation about three perpendicular axes, often termed yaw (normal axis), pitch (transverse axis), and roll (longitudinal axis). The processing unit may be configured to continuously model chest and/or abdominal movement based on the body surface images obtained from the surface camera system.

In one embodiment the processing unit is configured to continuously extract an amplitude of chest height movement during a respiratory cycle. The processing unit may, alternatively, or additionally, be configured to detect a deviation of height, shape or movement of the chest and/or abdomen of the patient. The extraction of height, shape or movement of the chest of the patient may be extracted from a model of the chest and/or abdominal movement, for example, from continuously modelled chest and/or abdominal movement in six degrees of freedom (6DOF).

In one embodiment the processing unit is configured to distinguish between patient movement and chest and/or abdominal movement. Based on the model of the surface, which may be a surface of the chest and/or abdominal region of the patient, or based on, for example a comparison with other modeled references of the patient, the system may distinguish between general patient movement and local chest and/or abdominal movement. The processing unit may be configured to distinguish between patient movement and chest and/or abdominal movement based on the modelled chest and/or abdominal movement of the patient. The processing unit may be configured to distinguish between patient movement and chest and/or abdominal movement based on the body surface images.

The feedback from the surface camera system can be used in a number of applications. In one embodiment a control unit is configured to optimize the continuous positive pressure to minimize the chest and/or abdominal movement of the patient based on the continuously model chest and/or abdominal movement of the patient. As would be recognized by a person skilled in the art, the control unit and the processing unit may be provided as separate devices or as one unit. The control unit and the processing unit may form part of any of the other parts of the system, such as the surface imaging system, the radiotherapy system and the positive pressure unit. The control unit may be configured to optimize the continuous positive pressure to minimize the chest and/or abdominal movement of the patient based on the body surface images. The control unit may be configured to optimize the continuous positive pressure to minimize the chest and/or abdominal movement of the patient based on the modelled chest and/or abdominal movement of the patient.

The hybrid continuous positive pressure and surface imaging system may be configured to continuously model chest and/or abdominal movement of the patient in substantially real-time. This can be done, for example, by obtaining body surface images of the patient, in particular body surface images of the chest and/or abdominal region of the patient, and using the body surface images in a physiological model of the patient to model the chest and/or abdominal movement. The body surface images may thus be translated to a model of a chest and/or abdominal volume using the physiological model of the patient.

The presently disclosed hybrid continuous positive pressure and surface imaging system may further comprise a control unit configured to control the continuous positive pressure to maintain the chest and/or abdominal movement within a predefined limit. The monitoring feedback mechanism may thus allow an operator to adjust the positive pressure to a point where chest movement is within defined limits, or minimised. The monitoring feedback mechanism may allow for automatic adjustment of the positive pressure to a point where chest movement is within defined limits, or minimised. This is normally done within the setup environment, but if excessive chest movement is detected in the treatment room the pressure may be adjusted to correct for this. In one embodiment the continuous positive pressure is gradually increased until the chest and/or abdominal movement is within the predefined limit. For example, the positive pressure can be automatically increased in a controlled fashion over a defined ramp period to avoid until consistent chest position is achieved or the positive pressure reaches a predefined limit. The control unit may be configured to control the continuous positive pressure to maintain the chest and/or abdominal movement within a predefined limit based on the body surface images. The control unit may be configured to control the continuous positive pressure to maintain the chest and/or abdominal movement within a predefined limit based on the modelled chest and/or abdominal movement of the patient.

The positive pressure unit preferably comprises an air pump unit and a flow tube. The air pump unit may comprise a blower. In one embodiment the positive pressure unit comprises an airway connected to the blower with integrated attachments for pressure and volume measurement and a nozzle for attachment of a hose. The PPU may further comprise a control unit configured to control the positive pressure unit. The control unit may be configured to control the positive pressure unit based on the body surface images. The control unit may be configured to control the positive pressure unit based on the modelled chest and/or abdominal movement of the patient. In one embodiment, the control unit provides a signal for adjustment of the continuous positive pressure provided by the positive pressure unit. For example, the speed of the blower may be controlled by Pulse Width Modulation (PWM) that allows fine speed adjustment, and provides a signal proportional to the fan speed. Other suitable method for controlling the positive pressure unit are possible. The PPU may detect variations in pressure and adjust fan speed accordingly to maintain the desired pressure.

The positive pressure unit may be configured to apply air pressure to a patient's airway to expand the lungs and raise the chest and minimises chest movement during the radiotherapy treatment process. This offers the benefits of stabilising the position of the chest and at the same time moving the heart away from the left breast. This also increases lung volume redistributing normal lung tissue away from the dose and reduces tumor motion in the lung. During radiotherapy this has the benefit of reducing unintended lung and cardiac dose.

The positive pressure unit may be configured to perform a procedure at a target site in the thoracic or abdominal regions of a patient's body and constrain movement of the target site due to respiratory motion during the procedure. The positive pressure unit may be configured to provide continuous positive airway pressure to the patient's lungs during and/or prior to a radiotherapy procedure to constrain motion of the target site, expand the chest cavity and lungs, and/or displace organs and tissue in the chest cavity to distance them from the target site.

The hybrid continuous positive pressure and surface imaging system may further comprise a pressure measurement unit for measuring a lung pressure. The processing unit may be configured to compare a measured lung pressure against an expected lung pressure. The processing unit may be further configured to verify a mismatch between the measured lung pressure and the expected lung pressure against the continuously modelled chest and/or abdominal movement. If there is a mismatch between measured pressure and expected measurements an error condition can be flagged. This can be signalled to the control unit, which may notify the operator. Similarly a beam hold can be signalled to the radiotherapy system. The measurement unit may be further adapted to measure lung volume and/or from around the mask or in the connection to the mask. The positive pressure unit may further comprise a power source, such as a rechargeable battery, and a wireless communication unit for communication, directly or through a further unit, with the surface camera system. The PPU may be a portable PPU.

The presently disclosed hybrid continuous positive pressure and surface imaging system may be configured to store patient specific pressure settings for individual patients. This may allow reproducibility of the respiratory patterns. The disclosed hybrid continuous positive pressure may continuously measure and/or monitor a number of parameters. If, for example, a measured pressure is outside a predefined tolerance, or a tidal volume exceeds a defined tolerance, an error condition may be flagged. The hybrid continuous positive pressure and surface imaging system may comprise a watchdog, for example, in the form of an application running on the processing unit checking the pressure of the PPU or error conditions from the PPU at intervals. When transitioning between setup and treatment rooms the watchdog may be disabled for a preset period.

The CPAP may be configured to operate to flatten and reduce motion of the diaphragm during respiration, and expand the lungs and chest cavity. The CPAP may thereby moderate motion of tissue and organs in the thoracic and abdominal regions due to patient respiration, and, as a result, motion of the target of the radiotherapy system during irradiation. Expansion of the lungs and chest cavity tends to reposition and increase spacing between organs in the chest cavity and abdomen. The reduced motion of the target site and other organs and tissue in the thoracic and abdominal regions of the patient facilitates delivery of a medical device or agent to the target site and contributes to reduction of possible collateral damage to tissue outside of the target site resulting from delivery of the medical device or agent. Expansion of the lungs and chest cavity and resultant repositioning and spacing of organs in the chest and abdomen may also facilitate delivery of a medical device or agent to the target site and reduction of possible collateral damage to tissue outside of the target site resulting from delivery of the medical device or agent.

The surface camera system configured to continuously capture body surface images of the patient may comprise one or more three-dimensional camera(s) and/or one or more two-dimensional camera(s). The processing unit may be configured to continuously model chest and/or abdominal movement of the patient based on the body surface images from the camera(s). There are several possible techniques and camera systems that can be used as surface camera system, including systems provided by Vision RT.

The surface camera system may, according to a non-limiting example, be adapted to provide stereoscopic body surface images of the patient. Stereoscopic images of a patient may thereby be obtained and processed to generate data identifying 3D positions of a large number of points corresponding to points on the surface of an imaged patient.

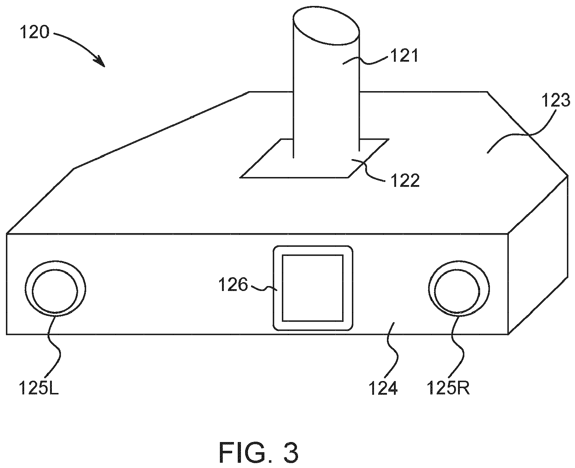

One example of an embodiment of a surface camera system is shown in fig. 3. Fig. 3 is a front perspective view of an exemplary surface camera system 120. The surface camera system 120 in this example comprises a housing 123 which is connected to a bracket 121 via a hinge 122. The bracket 121 enables the surface camera system 120 to be attached in a fixed location to the ceiling of a treatment room whilst the hinge 122 permits the orientation of the surface camera system 120 to be orientated relative to the bracket 121 so that the surface camera system 120 can be arranged to view a patient 300 on a couch 150. A pair of lenses 125L/125R are mounted at either end of the front surface 124 of the housing of the surface camera system 120. These lenses 125L/125R may be positioned in front of image capture devices/cameras such as CMOS active pixel sensors or charge coupled devices (not shown) contained within the housing 123. The cameras/image detectors are preferably arranged behind the lenses 125L/125R so as to capture images of a patient 300 via the lenses 125U125R. In this example, a speckle projector 126 is provided in the middle of the front surface 124 of the housing 123 between the two lenses 125L/125R in the surface camera system 120 shown in fig. 3. The speckle projector 126 in this example is arranged to illuminate a patient 300 with a non-repeating speckled pattern of light, such as red light, so that when images of a patient 300 are captured by the two image detectors mounted within a surface camera system 120 corresponding portions of captured images can be more easily distinguished. To that end the speckle projector comprises a light source such as a LED and a film with a random speckle pattern printed on the film. In use light from the light source is projected via the film and as a result a pattern consisting of light and dark areas is projected onto the surface of a patient 300. In some monitoring systems, the speckle projector 126 could be replaced with a projector arranged to project structured light (e.g. laser light) in the form of a line or a grid pattern onto the surface of a patient 300.

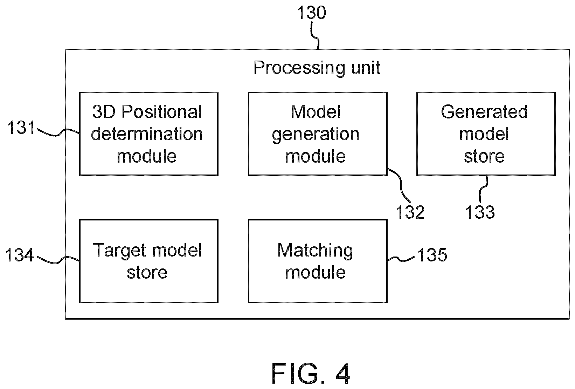

Fig. 4 is a schematic block diagram of an example of the processing unit 130. In order for the processing unit 130 to process images received from the surface camera system 120, the processing unit 130 is configured by software either provided on a disk or by receiving an electrical signal via a communications network into a number of functional modules 131-135. In this example, the functional modules 131-135 comprise: a 3D position determination module 131 for processing images received from the surface camera system 120; a model generation module 132 for processing data generated by the 3D position determination module 131 and converting the data into a 3D wire mesh model of an imaged computer surface; a generated model store 133 for storing a 3D wire mesh model of an imaged surface; a target model store 134 for storing a previously generated 3D wire mesh model; and a matching module 135 for determining rotations and translations required to match a generated model with a target model. In use, as images are obtained by the surface camera system 120, these images are processed by the 3D position determination module 131. This processing enables the 3D position determination module 131 to identify 3D positions of corresponding points in pairs of images on the surface of a patient 300. In the exemplary system, this is achieved by the 3D position determination module 131 identifying corresponding points in pairs of images obtained by the surface camera system and then determining 3D positions for those points based on the relative positions of corresponding points in obtained pairs of images and stored camera parameters for the surface camera system 120.

The presently disclosed hybrid continuous positive pressure and surface imaging system is not limited to the above example of surface camera system 120 and the processing unit 130. A further example of a surface imaging system comprises a sheet that can be placed on the patient, for example, placed on a chest and/or abdominal surface of the patient. Preferably, at least a portion of the sheet is colored. The surface camera system may be operable to obtain images of the patient and the sheet. A model generation module may be operable to process images obtained by the surface camera system to generate a model of the surface of a portion of a patient appearing in the images. In some embodiments the system may additionally comprise a projector operable to project a pattern of light onto a patient. In such embodiments, preferably, the colored portion of the sheet is arranged to absorb a substantial proportion of the projected light projected by the projector so that the projected pattern is not readily apparent in the portion of the images corresponding to the sheet. This may be achieved by the color of the sheet being substantially black so as to absorb a substantial proportion of the projected light. In other embodiments the color of the sheet may be selected so as to correspond to a color associated with the wavelength of the projected light so that the projection of the light onto the surface of the sheet is not readily apparent. In some embodiments the sheet may be colored black.

The surface camera system may be adapted to provide stereoscopic body surface images of the patient. The processing unit may accordingly be configured to process the stereoscopic body surface images and generate a model of the surface of the chest of the patient.

The radiotherapy system may be a conventional two-dimensional beam system but could also be, for example, a three-dimensional beam system, an intensity-modulated radiation therapy (IMRT) system or a volumetric modulated arc therapy (VMAT) system. The radiotherapy system may be an external beam radiation therapy system. There are a number of techniques available, including conventional external beam radiation therapy using medical linear accelerators. Typically the treatment is simulated prior to the treatment. The aim of simulation is to accurately target or localize the volume which is to be treated. This technique is well established and is generally quick and reliable. A challenge is that some high-dose treatments may be limited by the radiation toxicity capacity of healthy tissues which lie close to the target tumor volume. Other types of radiotherapy systems are stereotactic radiation, intensity-modulated radiation therapy and volumetric modulated arc therapy. The presently hybrid continuous positive pressure and surface imaging system is not limited to any particular type of radiotherapy system. The radiotherapy system may typically include a radiation beam generator adapted to generate a radiation beam for irradiating a target volume.

It is possible to use further systems, such as a fluoroscopy-based system, for tracking tumor motion.

A control unit may be configured to discontinue operation of the radiation beam upon a deviation of an extracted height or shape of the chest and/or abdomen of the patient with respect to an expected height or shape of the chest and/or abdomen of the patient. In a further embodiment the control unit is configured to synchronize the radiation beam with the extracted height or shape of the chest of the patient.

The disclosure further relates to a computer-implemented method of providing real-time feedback to a positive pressure unit, the method comprising the steps of:
acquiring continuous positive pressure data from continuous positive pressure applied to lungs of a patient during radiotherapy; continuously capturing body surface images of the patient using a surface camera system; and continuously modelling chest and/or
abdominal movement of the patient in response to the applied continuous positive pressure based on the body surface images.

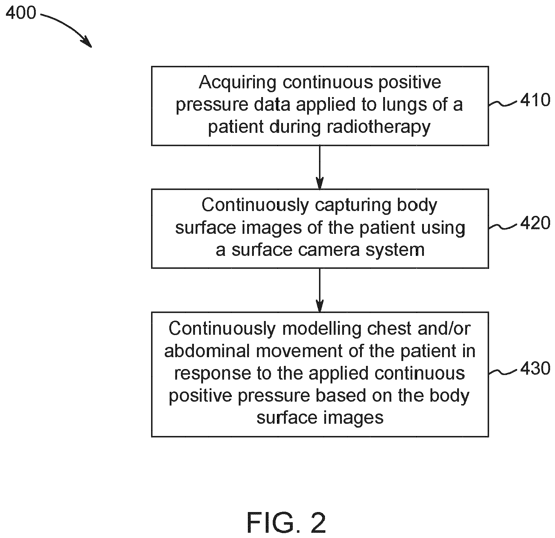

Fig. 2 shows a flow chart of a method according to embodiments of the presently disclosed computer-implemented method of providing real-time feedback to a positive pressure unit (400). The method comprises the steps of: acquiring continuous positive pressure data from continuous positive pressure applied to lungs of a patient during radiotherapy (410); continuously capturing body surface images of the patient using a surface camera system (420); and continuously modelling chest and/or abdominal movement of the patient in response to the applied continuous positive pressure based on the body surface images (430). The method may further comprise the step of applying a continuous positive pressure to lungs of a patient during radiotherapy. The method may further comprise the step of optimizing the continuous positive pressure to minimize the chest and/or abdominal movement of the patient and/or to control the continuous positive pressure to maintain the chest and/or abdominal movement within a predefined limit.

The method may be implemented as a computer program having instructions, which, when executed by a computing device or computing system, cause the computing device or computing system to carry out the method of providing real-time feedback to a positive pressure unit. A non-transitory storage medium may comprise a computer program product having instructions embodied thereon, the computer program product, when executed by a computing device or system, causes the computing device or system to provide real-time feedback to a positive pressure unit.

## Claims

1. A hybrid continuous positive pressure and surface imaging system for use in a radiotherapy system, comprising:
a positive pressure unit adapted to apply a continuous positive pressure to lungs of a patient during radiotherapy;
a surface camera system configured to continuously, or at intervals, capture body surface images of the patient; and
a processing unit configured to continuously, or at intervals, model chest and/or abdominal movement of the patient, in response to the applied continuous positive pressure, based on the body surface images.

2. The hybrid continuous positive pressure and surface imaging system according to claim 1, wherein the processing unit is configured to continuously, or at intervals, extract an amplitude metric translatable to chest and/or abdominal movement.

3. The hybrid continuous positive pressure and surface imaging system according to any one of the preceding claims, wherein the processing unit is configured to continuously, or at intervals, extract a height of the chest wall and/or abdomen of the patient.

4. The hybrid continuous positive pressure and surface imaging system according to any one of the preceding claims, wherein the processing unit is configured to continuously, or at intervals, model chest and/or abdominal movement in six degrees of freedom (6DOF).

5. The hybrid continuous positive pressure and surface imaging system according to any one of the preceding claims, wherein the processing unit is configured to extract a change of an amplitude of chest and/or abdominal height movement during a respiratory cycle.

6. The hybrid continuous positive pressure and surface imaging system according to any one of the preceding claims, wherein the processing unit is configured to detect a deviation of height, shape or movement of the chest and/or abdomen of the patient.

7. The hybrid continuous positive pressure and surface imaging system according to any one of the preceding claims, wherein the processing unit is configured to distinguish between patient movement and chest and/or abdominal movement based on the body surface images.

8. The hybrid continuous positive pressure and surface imaging system according to any one of the preceding claims, further comprising a control unit configured to optimize the continuous positive pressure to minimize the chest and/or abdominal movement of the patient based on the body surface images.

9. The hybrid continuous positive pressure and surface imaging system according to any one of the preceding claims, wherein the processing unit is configured to continuously, or at intervals, model chest and/or abdominal movement of the patient in substantially real-time.

10. The hybrid continuous positive pressure and surface imaging system according to any one of the preceding claims, further comprising a control unit configured to control the continuous positive pressure to maintain the chest and/or abdominal movement within a predefined limit based on the body surface images.

11. The hybrid continuous positive pressure and surface imaging system according to claim 10, wherein the continuous positive pressure is gradually increased until the chest and/or abdominal movement is within the predefined limit.

12. The hybrid continuous positive pressure and surface imaging system according to any one of the preceding claims, wherein the positive pressure unit comprises an air pump unit and a flow tube.

13. The hybrid continuous positive pressure and surface imaging system according to claim 12, wherein the air pump unit comprises a blower.

14. The hybrid continuous positive pressure and surface imaging system according to any one of the preceding claims, further comprising a control unit configured to control the positive pressure unit based on the body surface images.

15. The hybrid continuous positive pressure and surface imaging system according to claim 14 and any one of claims 12-13, wherein the control unit provides a signal for adjustment of the continuous positive pressure provided by the positive pressure unit.

16. The hybrid continuous positive pressure and surface imaging system according to any one of the preceding claims, further comprising a pressure measurement unit for measuring a lung pressure.

17. The hybrid continuous positive pressure and surface imaging system according to claim 16, wherein the processing unit is configured to compare a measured lung pressure against an expected lung pressure, and wherein the processing unit is further configured to verify a mismatch between the measured lung pressure and the expected lung pressure against the continuously modelled chest and/or abdominal movement.

18. The hybrid continuous positive pressure and surface imaging system according to any one of the preceding claims, wherein the surface camera system is adapted to provide stereoscopic body surface images of the patient.

19. The hybrid continuous positive pressure and surface imaging system according to claim 18, wherein the processing unit is configured to process the stereoscopic body surface images and generate a model of the surface of the chest and/or abdomen of the patient.

20. The hybrid continuous positive pressure and surface imaging system according to any one of the preceding claims, further comprising a radiation beam generator, such as a gated radiation beam generator, adapted to generate a radiation beam for irradiating a target volume.

21. The hybrid continuous positive pressure and surface imaging system according to claim 20, wherein a control unit is configured to discontinue operation of the radiation beam upon a deviation of height, shape or movement of the chest and/or abdomen of the patient with respect to an expected height, shape or movement of the chest and/or abdomen of the patient or a deviation in surface caused by the change in lung volume.

22. The hybrid continuous positive pressure and surface imaging system according to any one of claims 20-21, wherein a control unit is configured to synchronize the radiation beam with the extracted height or shape of the chest and/or abdomen of the patient.

23. The hybrid continuous positive pressure and surface imaging system according to claim 20 and claim 17, wherein a control unit is configured to discontinue operation of the radiation beam if a mismatch occurs.
